# EUROPEAN PATENT SPECIFICATION

(11) **EP 2 780 314 B1**
(45) Date of publication and mention of the grant of the patent: **20.12.2017**
(21) Application number: 12849256.8
(22) Date of filing: 05.11.2012
(51) Int. Cl.: C07C 67/54, C07C 69/704, B01D 15/08, C07C 67/08, A24B 15/24

(54) **METHOD FOR PRODUCING TRIETHYL CITRATE FROM TOBACCO**
VERFAHREN ZUR HERSTELLUNG VON TRIETHYLCITRAT AUS TABAK
PROCÉDÉ DE PRODUCTION DE CITRATE DE TRIÉTHYLE À PARTIR DE TABAC

(30) Priority: 17.11.2011 US 201113298602
(43) Date of publication of application: 24.09.2014
(73) Proprietor: R. J. Reynolds Tobacco Company, Winston-Salem, North Carolina 27101-3804 (US)
(72) Inventor: DUBE, Michael Francis, Winston-Salem North Carolina 27104 (US); COLEMAN, William, Winston-Salem North Carolina 27106 (US)
(74) Representative: Wilkins, Christopher George
(86) International application number: PCT/US2012/063510
(87) International publication number: WO 2013/074315

(56) References cited:
- WO-A1-98/51657
- US-A1- 2002 197 688
- KOLAH ET AL.: 'Reaction Kinetics of the Catalytic Esterification of Citric Acid with Ethanol' IND.ENG.CHEM.RES. vol. 46, 2007, pages 3180 - 3187, XP002448003
- VICKERY ET AL.: 'THE NON-VOLATILE ORGANIC ACIDS OF GREEN TOBACCO LEAVES' JOURNAL OF BIOLOGICAL CHEMISTRY vol. 90, 31 March 1931, pages 637 - 653, XP055069352
- CLARK T. JEFFREY ET AL.: 'Derivatization Solid-Phase Micro extraction Gas Chromatographic-Mass Spectrometric Determination of Organic Acids in Tobacco' JOURNAL OF CHROMATOGRAPHIC SCIENCE vol. 35, no. 5, 1997, pages 209 - 212, XP055069353
- SADECKA J. ET AL.: 'Determination of organic acids in tobacco by capillary isotachophoresis' JOURNAL OF CHROMATOGRAPHY A vol. 988, 2003, pages 161 - 165, XP055069354

## Description

### FIELD OF THE INVENTION

The present invention relates to products made or derived from tobacco or, more generally, made or derived from any biomass derived from any one or more species of genus *Nicotiana,* or that otherwise incorporate tobacco, and are intended for human consumption. Of particular interest are ingredients or components obtained or derived from plants or portions of plants from the *Nicotiana* species.

### BACKGROUND OF THE INVENTION

Popular smoking articles, such as cigarettes, have a substantially cylindrical rod shaped structure and include a charge, roll or column of smokable material such as shredded tobacco (e.g., in cut filler form) surrounded by a paper wrapper thereby forming a so-called "tobacco rod." Normally, a cigarette has a cylindrical filter element aligned in an end-to-end relationship with the tobacco rod. Typically, a filter element comprises plasticized cellulose acetate tow circumscribed by a paper material known as "plug wrap." Certain cigarettes incorporate a filter element having multiple segments, and one of those segments can comprise activated charcoal particles. Typically, the filter element is attached to one end of the tobacco rod using a circumscribing wrapping material known as "tipping paper." It also has become desirable to perforate the tipping material and plug wrap, in order to provide dilution of drawn mainstream smoke with ambient air. A cigarette is employed by a smoker by lighting one end thereof and burning the tobacco rod. The smoker then receives mainstream smoke into his/her mouth by drawing on the opposite end (e.g., the filter end) of the cigarette.

The tobacco used for cigarette manufacture is typically used in blended form. For example, certain popular tobacco blends, commonly referred to as "American blends," comprise mixtures of flue-cured tobacco, burley tobacco, and Oriental tobacco, and in many cases, certain processed tobaccos, such as reconstituted tobacco and processed tobacco stems. The precise amount of each type of tobacco within a tobacco blend used for the manufacture of a particular cigarette brand varies from brand to brand. However, for many tobacco blends, flue-cured tobacco makes up a relatively large proportion of the blend, while Oriental tobacco makes up a relatively small proportion of the blend. See, for example, Tobacco Encyclopedia, Voges (Ed.) p. 44-45 (1984), Browne, The Design of Cigarettes, 3rd Ed., p. 43 (1990) and Tobacco Production, Chemistry and Technology, Davis et al. (Eds.) p. 346 (1999).

Through the years, various treatment methods and additives have been proposed for altering the overall character or nature of tobacco materials utilized in tobacco products. For example, additives or treatment processes have been utilized in order to alter the chemistry or sensory properties of the tobacco material, or in the case of smokable tobacco materials, to alter the chemistry or sensory properties of mainstream smoke generated by smoking articles including the tobacco material. The sensory attributes of cigarette smoke can be enhanced by incorporating flavoring materials into various components of a cigarette. Exemplary flavoring additives include menthol and products of Maillard reactions, such as pyrazines, aminosugars, and Amadori compounds. See also, Leffingwell et al., Tobacco Flavoring for Smoking Products, R.J. Reynolds Tobacco Company (1972). In some cases, treatment processes involving the use of heat can impart to the processed tobacco a desired color or visual character, desired sensory properties, or a desired physical nature or texture. Various processes for preparing flavorful and aromatic compositions for use in tobacco compositions are set forth in US Pat. Nos. 3,424,171 to Rooker; 3,476,118 to Luttich; 4,150,677 to Osborne, Jr. et al.; 4,986,286 to Roberts et al.; 5,074,319 to White et al.; 5,099,862 to White et al.; 5,235,992 to Sensabaugh, Jr.; 5,301,694 to Raymond et al.; 6,298,858 to Coleman, III et al.; 6,325,860 to Coleman, III et al.; 6,428,624 to Coleman, III et al.; 6,440,223 to Dube et al.; 6,499,489 to Coleman, III; and 6,591,841 to White et al.; US Pat. Appl. Publication No. 2004/0173228 to Coleman, III; and US Appl. Serial No. 12/191,751 to Coleman, III et al., filed August 14, 2008. Additionally, examples of representative components that can be employed as so-called natural tar diluents in tobacco products are set in PCT WO 2007/012980 to Lipowicz.

Tobacco also may be enjoyed in a so-called "smokeless" form. Particularly popular smokeless tobacco products are employed by inserting some form of processed tobacco or tobacco-containing formulation into the mouth of the user. Various types of smokeless tobacco products are set forth in US Pat. Nos. 1,376,586 to Schwartz; 3,696,917 to Levi; 4,513,756 to Pittman et al.; 4,528,993 to Sensabaugh, Jr. et al.; 4,624,269 to Story et al.; 4,987,907 to Townsend; 5,092,352 to Sprinkle, III et al.; and 5,387,416 to White et al.; US Pat. Appl. Pub. Nos. 2005/0244521 to Strickland et al.; 2008/0196730 to Engstrom et al.; and 2009/0293889 to Kumar et al.; PCT WO 04/095959 to Arnarp et al.; PCT WO 05/063060 to Atchley et al.; PCT WO 05/016036 to Bjorkholm; and PCT WO 05/041699 to Quinter et al. See, for example, the types of smokeless tobacco formulations, ingredients, and processing methodologies set forth in US Pat. Nos. 6,953,040 to Atchley et al. and 7,032,601 to Atchley et al.

One type of smokeless tobacco product is referred to as "snuff." Representative types of moist snuff products, commonly referred to as "snus," have been manufactured in Europe, particularly in Sweden, by or through companies such as Swedish Match AB, Fiedler & Lundgren AB, Gustavus AB, Skandinavisk Tobakskompagni A/S, and Rocker Production AB. Snus products available in the U.S.A. have been marketed under the tradenames Camel Snus Frost, Camel Snus Original and Camel Snus Spice by R. J. Reynolds Tobacco Company. See also, for example, Bryzgalov et al., 1N1800 Life Cycle Assessment, Comparative Life Cycle Assessment of General Loose and Portion Snus (2005). In addition, certain quality standards associated with snus manufacture have been assembled as a so-called GothiaTek standard. Representative smokeless tobacco products also have been marketed under the tradenames Oliver Twist by House of Oliver Twist A/S; Copenhagen, Skoal, SkoalDry, Rooster, Red Seal, Husky, and Revel by U.S. Smokeless Tobacco Co.; "taboka" by Philip Morris USA; Levi Garrett, Peachy, Taylor's Pride, Kodiak, Hawken Wintergreen, Grizzly, Dental, Kentucky King, and Mammoth Cave by Conwood Company, LLC; and Camel Orbs, Camel Sticks, and Camel Strips by R. J. Reynolds Tobacco Company.

The sensory attributes of smokeless tobacco can also be enhanced by incorporation of certain flavoring materials. See, for example, US Pat. Appl. Pub. Nos. 2002/0162562 to Williams; 2002/0162563 to Williams; 2003/0070687 to Atchley et al.; 2004/0020503 to Williams, 2005/0178398 to Breslin et al.; 2006/0191548 to Strickland et al.; 2007/0062549 to Holton, Jr. et al.; 2007/0186941 to Holton, Jr. et al.; 2007/0186942 to Strickland et al.; 2008/0029110 to Dube et al.; 2008/0029116 to Robinson et al.; 2008/0029117 to Mua et al.; 2008/0173317 to Robinson et al.; and 2008/0209586 to Neilsen et al.

Found as an ingredient in any of a number of tobacco product formulations is triethyl citrate (CAS 102-76-1). Triethyl citrate is also known as citric acid, ethyl ester; it is also referred to as triethylis citras. While it may serve any of a number of functions in tobacco product formulations, triethyl citrate is known in the art to serve as a plasticizer. In addition, triethyl citrate may function as a flavorant or a surfactant. A clear, oily liquid at typical room temperature, triethyl citrate is odorless and practically colorless.

Triethyl citrate is useful as a component of a variety of tobacco products or as an ingredient in the processing of tobacco. See, for example, US Pat. Nos. 4,007,745 to Randall and Keith; 4,007,746 to Sawada and Kotani; 4,522,616 to Howell et al.; 5,101,839 to Jakob et al.; 5,105,836 to Gentry et al.; 5,105,838 to White and Perfetti; 5,129,408 to Jakob et al.; 5,598,868 to Jakob et al.; 5,706,833 to Tsuyaga et al.; 5,758,669 to Taniguchi and Nishimura; 5,947,127 to Tsugaya et al.; 6,095,152 to Beven et al.; 6,289,897 to McAdam et al.; 6,397,852 and 6, 408, 856 to McAdam; 6,578,584 to Beven et al.; and 7,938,125 to John and Sutton.

As it should be clear from the foregoing that triethyl citrate is useful in the formulation of various tobacco products, it can also be seen that it would accordingly be desirable to provide a method for producing triethyl citrate from tobacco, that is, in particular, from *Nicotiana* species, for use, *inter alia,* in tobacco compositions utilized in a variety of tobacco products or in the processing of tobacco.

### SUMMARY OF THE INVENTION

Materials from *Nicotiana* species (e.g., tobacco-derived materials) comprising isolated components from plants of the *Nicotiana* species useful for incorporation into tobacco compositions utilized in a variety of tobacco products, such as smoking articles and smokeless tobacco products are disclosed herein. Methods are disclosed herein for isolating components from *Nicotiana* species (e.g., tobacco materials), and for processing those components and tobacco materials incorporating those components. For example, tobacco-derived materials can be prepared by subjecting at least a portion of a tobacco plant (e.g., leaves, stalks, roots, or stems) to a separation process, which typically can include multiple sequential extraction steps, in order to isolate desired components of the tobacco material.

When used in connection with the invention, the term "biomass" denotes one or more portions of a plant, and in particular denotes substantially the entirety of the superterranean portion of a plant, optionally including some or all of the subterranean portion of a plant. Accordingly, the term "biomass" may refer to leaf or to seed or to any other superterranean portion of a plant, or to any combination thereof, optionally including some or all of the subterranean portion of a plant.

When used in connection with the invention, the term "one or more plants of genus *Nicotiana"* denotes any one or more plants of the genus *Nicotiana* of family *Solanaceae,* including, for example, any one or more of the following: N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata, and N. x sanderae, N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. rustica, N. simulans, N. stocktonii, N. suaveolens, N. tabacum, N. umbratica, N. velutina, and N. wigandioides, N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis subsp. Hersperis, N. pauciflora, N. petunioides, N. quadrivalvis, N. repanda, N. rotundifolia, N. Solanifolia, N. spegazzinii.

The use of *Nicotiana*-derived (e.g., tobacco-derived) materials disclosed herein enables the preparation of tobacco compositions for smoking articles or smokeless tobacco compositions that are derived substantially or even entirely from *Nicotiana* materials. For example, a tobacco composition can incorporate tobacco or tobacco-derived material of some form, including isolated components from *Nicotiana* species, such that at least about 80 weight percent, more typically at least about 90 weight percent, or even at least about 95 weight percent (on a dry weight basis), of that tobacco composition consists of tobacco-derived material.

It has been recognized that there is a need to make fuller use of material or substance from tobacco, and in particular from plants or portions of plants from the *Nicotiana* species. Readily available starting materials or inputs from plants or portions of plants from the *Nicotiana* species, such starting materials or inputs being useful in particular for inclusion as starting materials or inputs in a process whereby material or substance from tobacco can be more fully utilized, include inter alia tobacco biomass and tobacco seed. Tobacco biomass can include for example the entirety of the substance of a tobacco plant that has been harvested whole. Tobacco biomass can include for example essentially all of the superterranean parts of a tobacco plant and optionally can include some or all of the subterranean parts of a tobacco plant. Tobacco biomass can include for example the solid portion of a tobacco plant that has been harvested whole, or the solid portion of essentially all of superterranean parts of a tobacco plant, and from which so-called "green juice" has been expelled for example through the action of a screw press. Tobacco biomass can include for example such a solid portion from which at least a portion of the water has been removed by drying. A tobacco seed may occupy only a very small volume, such as a fraction of a cubic millimeter. For this reason, it is typically practical to collect a plurality of tobacco seeds when it is chosen to harvest tobacco seed.

Among ways in which fuller use can be made of material or substance from tobacco, and in particular from plants or portions of plants from the *Nicotiana* species, are various chemical transformations to which plants or portions of plants from the *Nicotiana* species can be subjected. Such chemical transformations may result in outputs or products having one or more desired or favorable properties. Such outputs or products may themselves be useful as starting material or inputs for further useful processes. Among chemical transformations to which plants or portions of plants from the *Nicotiana* species can be subjected are fermentations and extractions.

Various processes for fermentation of plant biomass are known in the art, among which are those set forth in U.S. Pat. Nos. 7,820,419 to Smith et al. and 7,943,350 to Vlasenko et al.; in U.S. Pat. Appl. Pub. Nos. 2002/0197688 of Pandolfino, 2010/0017916 of Pappan et al., and 2010/0196980 of Smith et al.; and in PCT WO 2002/098208 of Pandolfino and 2011/100272 of Wietgrefe and Bregger. A product of such a fermentation process may be ethanol.

Various processes for extraction, and in particular for extraction of one or more organic acids, and in further particular for citric acid, are known in the art, among which are those set forth in U.S. Pat. Nos. 4,251,671 to Alter and Blumberg; 4,334,095 to Baniel; and 5,426,220 to Baniel and Eyal. A product of such an extraction process may be citric acid.

Various processes for esterification, which is to say a condensation of an alcohol with an organic acid, are known in the art, among which are those, each embodying a synthesis of triethyl citrate, set forth in U.S. Pat Nos. 7,652,167 and 7,667,068 to Miller et al.; in U.S. Pat Appl. Pub. Nos. 2006/0014977 and 2006/0252956 of Miller et al.; and in Kolah et al. (2008), "Triethyl Citrate Synthesis by Reactive Distillation," Industrial and Engineering Chemistry Research, Vol. 47, No. 4, pp. 1017-1024.

It is known in the art that the intermediary metabolism of plants, including, *inter alia,* plants of genus *Nicotiana,* produces citric acid. See, for example, Shmuk and Pyatnitskii (1930), "Investigation of the Tobacco Acids," in Works of Academician A.A. Shmuk, Volume III, The Chemistry and Technology of Tobacco (Moscow: Pishchepromidzat, 1953; Jerusalem: trans. Lengy et al., Israel Program for Scientific Translations, 1961), pp. 136-144; Vickery and Pucher (1931), "The Non-Volatile Organic Acids of Green Tobacco Leaves," Journal of Biological Chemistry, Vol. 90, pp. 637-653; Shmuk (1933), "Tobacco and Makhorka As Raw Materials for the Production of Citric Acid," in Works, op. cit., pp. 688-707; Shmuk (1934), "The Method of Determination of Citric and Malic Acids in Tobacco and Makhorka," Ibid., pp. 247-251; Tso (1972), Physiology and Biochemistry of Tobacco Plants (Stroudsburg: Dowden, Hutchinson and Ross), p. 205. Accordingly, citric acid may readily be prepared from a plant source, such as from a plant source from any of the *Nicotiana* species.

A tobacco composition is disclosed herein for use in a smoking article or a smokeless tobacco composition comprising a tobacco material and a triethyl-citrate-containing component derived from biomass or a seed of the *Nicotiana* species, wherein the triethyl-citrate-containing component comprises triethyl citrate.

A citric-acid-containing component may be formed using distillation techniques adapted for obtaining citric acid from biomass or one or more anatomical parts of a plant. Alternatively, a citric-acid-containing component may be formed by extracting components from biomass or one or more anatomical parts of a plant using appropriate extraction techniques and solvents. Other separation processes can be used, such as chromatography, filtration, recrystallization, solvent-solvent partitioning, and combinations thereof. A citric-acid-containing component formed using an extraction process can be either the solvent-soluble portion or the insoluble residue of biomass or other plant material remaining after solvent extraction.

An ethanol-containing component may be formed using distillation techniques adapted for obtaining ethanol from biomass or one or more anatomical parts of a plant. Alternatively, an ethanol-containing component may be formed by extracting components from biomass or one or more anatomical parts of a plant using appropriate extraction techniques and solvents. Other separation processes can be used, such as chromatography, filtration, recrystallization, solvent-solvent partitioning, and combinations thereof. An ethanol-containing component formed using an extraction process can be either the solvent-soluble portion or the insoluble residue of biomass or other plant material remaining after solvent extraction.

An ethanol-containing component comprises ethanol, useful for the production of triethyl citrate. Likewise, a citric-acid-containing component comprises citric acid, also useful for the production of triethyl citrate. It will accordingly be seen that, when used in connection with the invention, an "citric-acid-containing component" may comprise citric acid or a salt of citric acid.

A triethyl-citrate-containing component can be used as such, or in the form of a chemically transformed triethyl-citrate-containing component. For example, a chemical transformation of a triethyl-citrate-containing component may include acid/base reaction, hydrolysis, thermal treatment, enzymatic treatment, and combinations of such steps.

A triethyl-citrate-containing component may be made from an ethanol-containing component derived from biomass or one or more anatomical parts of tobacco and a citric-acid-containing component derived from biomass or one or more anatomical parts of tobacco.

Smoking articles and smokeless tobacco compositions that include a triethyl-citrate-containing component are described herein. For example, a tobacco composition can incorporate a triethyl-citrate-containing component within a casing formulation or a top dressing formulation applied to tobacco strip or as a component of a reconstituted tobacco material.

The invention relates to a method for preparing triethyl citrate from one or more plants of the genus *Nicotiana* said method comprising (1) isolating an ethanol-containing component from harvested biomass or seed of the *Nicotiana* species by subjecting the harvested biomass or seed or a portion thereof to fermentation followed by cold pressing, solvent extraction, chromatography, distillation, filtration, recrystallization, solvent-solvent partitioning, or a combination thereof to form an isolated ethanol-containing component; (2) isolating a citric-acid-containing component from harvested biomass or one or more anatomical parts of the *Nicotiana* species by subjecting the harvested biomass or one or more anatomical parts or a portion thereof to cold pressing, solvent extraction, chromatography, distillation, filtration, recrystallization, solvent-solvent partitioning, or a combination thereof to form an isolated citric-acid-containing component; (3) reacting the ethanol-containing component with the citric-acid-containing component under conditions favoring esterification, thereby forming a triethyl-citrate-containing component. The method can further include the step of adding the triethyl-citrate-containing component to a tobacco composition adapted for use in a smoking article or a smokeless tobacco composition.

In connection with the invention it is accordingly found that a chemical transformation including a fermentation of plants or portions of plants from the *Nicotiana* species results in formation of a composition comprising ethanol and from which ethanol can be isolated; that a chemical transformation of plants or portions of plants from the *Nicotiana* species results in isolation of citric acid; and that an esterification reaction of ethanol so isolated and citric acid so isolated results in formation of triethyl citrate.

Triethyl citrate produced by the method of the invention is suitable for use in, on, or around a smoking article or a smokeless tobacco composition comprising a tobacco material and a component derived from the *Nicotiana* species, wherein the component is derived from the *Nicotiana* species.

### DETAILED DESCRIPTION OF THE PREFERRED EMBODIMENTS

The present invention now will be described more fully hereinafter. This invention may, however, be embodied in many different forms and should not be construed as limited to the embodiments set forth herein; rather, these embodiments are provided so that this disclosure will be thorough and complete, and will fully convey the scope of the invention to those skilled in the art. As used in this specification and the claims, the singular forms "a," "an," and "the" include plural referents unless the context clearly dictates otherwise. Reference to "dry weight percent" or "dry weight basis" refers to weight on the basis of dry ingredients (i.e., all ingredients except water).

The selection of the plant from the *Nicotiana* species can vary; and in particular, the types of tobacco or tobaccos may vary. Tobaccos that can be employed include flue-cured or Virginia (e.g., K326), burley, sun-cured (e.g., Indian Kurnool and Oriental tobaccos, including Katerini, Prelip, Komotini, Xanthi and Yambol tobaccos), Maryland, dark, dark-fired, dark air cured (e.g., Passanda, Cubano, Jatin and Bezuki tobaccos), light air cured (e.g., North Wisconsin and Galpao tobaccos), Indian air cured, Red Russian and *Rustica* tobaccos, as well as various other rare or specialty tobaccos. Descriptions of various types of tobaccos, growing practices and harvesting practices are set forth in Tobacco Production, Chemistry and Technology, Davis et al. (Eds.) (1999). Various representative types of plants from the *Nicotiana* species are set forth in Goodspeed, The Genus Nicotiana, (Chonica Botanica) (1954); US Pat. Nos. 4,660,577 to Sensabaugh, Jr. et al.; 5,387,416 to White et al. and 7,025,066 to Lawson et al.; US Patent Appl. Pub. Nos. 2006/0037623 to Lawrence, Jr. and 2008/0245377 to Marshall et al. Of particular interest are N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata, and N. x sanderae. Also of interest are N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. rustica, N. simulans, N. stocktonii, N. suaveolens, N. tabacum, N. umbratica, N. velutina, and N. wigandioides. Other plants from the *Nicotiana* species include N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis subsp. Hersperis, N. pauciflora, N. petunioides, N. quadrivalvis, N. repanda, N. rotundifolia, N. solanifolia and N. spegazzinii.

*Nicotiana* species can be derived using genetic-modification or crossbreeding techniques (e.g., tobacco plants can be genetically engineered or crossbred to increase or decrease production of certain components or to otherwise change certain characteristics or attributes). See, for example, the types of genetic modifications of plants set forth in US Pat. Nos. 5,539,093 to Fitzmaurice et al.; 5,668,295 to Wahab et al.; 5,705,624 to Fitzmaurice et al.; 5,844,119 to Weigl; 6,730,832 to Dominguez et al.; 7,173,170 to Liu et al.; 7,208,659 to Colliver et al.; and 7,230,160 to Benning et al.; US Patent Appl. Pub. No. 2006/0236434 to Conkling et al.; and PCT WO 2008/103935 to Nielsen et al.

For the preparation of smokeless and smokable tobacco products, it is typical for harvested plants of the *Nicotiana* species to be subjected to a curing process. Descriptions of various types of curing processes for various types of tobaccos are set forth in Tobacco Production, Chemistry and Technology, Davis et al. (Eds.) (1999). Exemplary techniques and conditions for curing flue-cured tobacco are set forth in Nestor et al., Beitrage Tabakforsch. Int., 20, 467-475 (2003) and U.S. Pat. No. 6,895,974 to Peele. See, also, for example, US Pat. No. 7,650,892 to Groves et al. Representative techniques and conditions for air curing tobacco are set forth in Roton et al., Beitrage Tabakforsch. Int., 21, 305-320 (2005) and Staaf et al., Beitrage Tabakforsch. Int., 21, 321-330 (2005). Certain types of tobaccos can be subjected to alternative types of curing processes, such as fire curing or sun curing. Preferably, harvested tobaccos that are cured are then aged.

At least a portion of the plant of the *Nicotiana* species (e.g., at least a portion of the tobacco portion) can be employed in an immature form. That is, the plant, or at least one portion of that plant, can be harvested before reaching a stage normally regarded as ripe or mature. As such, for example, tobacco can be harvested when the tobacco plant is at the point of a sprout, is commencing leaf formation, is commencing seeding, is commencing flowering, or the like.

At least a portion of the plant of the *Nicotiana* species (e.g., at least a portion of the tobacco portion) can be employed in a mature form. That is, the plant, or at least one portion of that plant, can be harvested when that plant (or plant portion) reaches a point that is traditionally viewed as being ripe, over-ripe or mature. As such, for example, through the use of tobacco harvesting techniques conventionally employed by farmers, Oriental tobacco plants can be harvested, burley tobacco plants can be harvested, or Virginia tobacco leaves can be harvested or primed by stalk position. After harvest, the plant of the *Nicotiana* species, or portion thereof, can be used in a green form (e.g., tobacco can be used without being subjected to any curing process). For example, tobacco in green form can be frozen, freeze-dried, subjected to irradiation, yellowed, dried, cooked (e.g., roasted, fried or boiled), or otherwise subjected to storage or treatment for later use. Such tobacco also can be subjected to aging conditions.

A tobacco product incorporates tobacco that is combined with some form of biomass or one or more anatomical parts obtained from, or derived from, a plant of at least one *Nicotiana* species. That is, a portion of a tobacco product can be composed of some form of biomass or one or more anatomical parts of a *Nicotiana* species, such as parts or pieces of biomass or one or more anatomical parts, or processed materials incorporating processed biomass or one or more anatomical parts or components thereof. At least a portion of the tobacco product can be composed of components of biomass or one or more anatomical parts, such as ingredients removed from biomass or one or more anatomical parts (e.g., by extraction, distillation, or other types of processing techniques). At least a portion of the tobacco product can be composed of components derived from biomass or one or more anatomical parts, such as components collected after subjecting biomass or one or more anatomical parts to chemical reaction or after subjecting components collected from biomass or one or more anatomical parts to chemical reaction (e.g., acid/base reaction conditions or enzymatic treatment).

The *Nicotiana* species can be selected for the type of biomass or anatomical part that it produces. For example, plants can be selected on the basis that those plants produce relatively abundant biomass or seed, produce biomass or seed that incorporate relatively high levels of specific desired components, and the like.

The *Nicotiana* species of plant can be grown under agronomic conditions so as to promote development of biomass or one or more anatomical parts. Tobacco plants can be grown in greenhouses, growth chambers, or outdoors in fields, or grown hydroponically.

Biomass or one or more anatomical parts are harvested from the *Nicotiana* species of plant. The manner by which biomass or one or more anatomical parts are harvested can vary. Typically, essentially all the biomass or anatomical parts can be harvested, and employed as such.

The time of harvest during the life cycle of the plant can vary. For example, biomass or one or more anatomical parts can be harvested when immature. Alternatively, biomass or one or more anatomical parts can be harvested after the point that the plant has reached maturity.

The post-harvest processing of biomass or one or more anatomical parts can vary. After harvest, the biomass or one or more anatomical parts, or portion thereof, can be used in the harvested form (e.g., the biomass can be used without being subjected to any curing and/or aging process steps). For example, biomass or one or more anatomical parts can be used without being subjected to significant storage, handling or processing conditions. In certain situations, it is preferable that the fresh biomass or one or more anatomical parts be used virtually immediately after harvest. Alternatively, for example, biomass or one or more anatomical parts can be refrigerated or frozen for later use, freeze dried, subjected to irradiation, yellowed, dried, cured (e.g., using air drying techniques or techniques that employ application of heat), heated or cooked (e.g., roasted, fried or boiled), or otherwise subjected to storage or treatment for later use.

Harvested biomass or seed can be physically processed. Biomass or one or more anatomical parts, or one or more parts thereof, can be further subdivided into parts or pieces (e.g., biomass or seed can be comminuted, pulverized, milled or ground into pieces or parts that can be characterized as granules, particulates or fine powders). Biomass or one or more anatomical parts, or one or more parts thereof, can be subjected to external forces or pressure (e.g., by being pressed or subjected to roll treatment). When carrying out such processing conditions, biomass or one or more anatomical parts can have a moisture content that approximates its natural moisture content (e.g., its moisture content immediately upon harvest), a moisture content achieved by adding moisture to the biomass or a moisture content that results from the drying of the biomass. For example, powdered, pulverized, ground or milled pieces of biomass or one or more anatomical parts can have moisture contents of less than about 25 weight percent, often less than about 20 weight percent, and frequently less than about 15 weight percent. Parts or pieces of biomass or one or more anatomical parts can be used as components of tobacco products without further processing, or alternatively the particulate biomass or anatomical part material can be processed further prior to incorporation into a tobacco product.

Harvested biomass or one or more anatomical parts, or components thereof, can be subjected to other types of processing conditions. For example, components of biomass or one or more anatomical parts can be separated from one another, or otherwise fractionated into chemical classes or mixtures of individual compounds. As used herein, an "isolated biomass component," "isolated component of one or more anatomical parts," "biomass isolate," or "isolate of one or more anatomical parts" is a compound or complex mixture of compounds separated from biomass or one or more anatomical parts of a plant of the *Nicotiana* species. The isolated biomass component or isolated component of one or more anatomical parts can be a single compound, a homologous mixture of similar compounds (e.g., isomers of a flavorful or aromatic compound), or a heterologous mixture of dissimilar compounds (e.g., a complex mixture of various compounds of different types, preferably having desirable sensory attributes).

Examples of the types of components that can be present in a biomass isolate or an isolate of one or more anatomical parts include various fatty acids and various triglycerides. Exemplary fatty acids include palmitic acid, linoleic acid, oleic acid, caprylic acid, myristic acid, pentadecanoic acid, palmetoleic acid, heptadecanoic acid, heptadecenoic acid, elaidic acid, gamma-lenolenic acid, arachidic acid, arachidonic acid, 11-eicosenoic acid, 8,11,14-eicosatrieonic acid, 11, 14,17-eicosatrienoic acid, 5,8,11,14,17-eicosopentanoic acid, heniecosenoic acid, lignoceric acid, 4,7,10,15,19- decosahexanoic acid, and stearic acid. Exemplary triglycerides include trilinolein, palmito-di-linolein, di-palmito-linolein, tripalmitin, tristearin, and triolein. Exemplary components of a biomass isolate or an isolate of one or more anatomical parts also include a variety of other compounds having flavor and aroma characteristics such as amino acids and various polyphenols.

Typical separation processes can include one or more process steps such as solvent extraction (e.g., using polar solvents, non-polar organic solvents, or supercritical fluids), chromatography, distillation, filtration, cold pressing or other pressure-based techniques, recrystallization, and/or solvent-solvent partitioning. Exemplary extraction and separation solvents or carriers include water, alcohols (e.g., methanol or ethanol), hydrocarbons (e.g., heptane and hexane), diethyl ether methylene chloride and supercritical carbon dioxide. Exemplary techniques useful for extracting components from *Nicotiana* species are described in US Pat. Nos. 4,144,895 to Fiore; 4,150,677 to Osborne, Jr. et al.; 4,267,847 to Reid; 4,289,147 to Wildman et al.; 4,351,346 to Brummer et al.; 4,359,059 to Brummer et al.; 4,506,682 to Muller; 4,589,428 to Keritsis; 4,605,016 to Soga et al.; 4,716,911 to Poulose et al.; 4,727,889 to Niven, Jr. et al.; 4,887,618 to Bernasek et al.; 4,941,484 to Clapp et al.; 4,967,771 to Fagg et al.; 4,986,286 to Roberts et al.; 5,005,593 to Fagg et al.; 5,018,540 to Grubbs et al.; 5,060,669 to White et al.; 5,065,775 to Fagg; 5,074,319 to White et al.; 5,099,862 to White et al.; 5,121,757 to White et al.; 5,131,414 to Fagg; 5,131,415 to Munoz et al.; 5,148,819 to Fagg; 5,197,494 to Kramer; 5,230,354 to Smith et al.; 5,234,008 to Fagg; 5,243,999 to Smith; 5,301,694 to Raymond et al.; 5,318,050 to Gonzalez-Parra et al.; 5,343,879 to Teague; 5,360,022 to Newton; 5,435,325 to Clapp et al.; 5,445,169 to Brinkley et al.; 6,131,584 to Lauterbach; 6,298,859 to Kierulff et al.; 6,772,767 to Mua et al.; and 7,337,782 to Thompson. See also, the types of separation techniques set forth in Brandt et al., LC-GC Europe, p. 2-5 (March, 2002) and Wellings, A Practical Handbook of Preparative HPLC (2006). In addition, the biomass or components thereof can be subjected to the types of treatments set forth in Ishikawa et al., Chem. Pharm. Bull., 50, 501-507 (2002); Tienpont et al., Anal. Bioanal. Chem., 373, 46-55 (2002); Ochiai, Gerstel Solutions Worldwide, 6, 17-19 (2006); Coleman, III, et al., J. Sci. Food and Agric., 84, 1223-1228 (2004); Coleman, III et al., J. Sci. Food and Agric., 85, 2645-2654 (2005); Pawliszyn, ed., Applications of Solid Phase Microextraction, RSC Chromatography Monographs, (Royal Society of Chemistry, UK) (1999); Sahraoui et al., J Chrom., 1210, 229-233 (2008); and 5,301,694 to Raymond et al. See also, for example, the types of processing techniques set forth in Frega et al., JAOCS, 68, 29-33 (1991); Patel et al., Tob. Res., 24, 44-49 (1998); Giannelos et al., Ind. Crops Prod., 16, 1-9 (2002); Mukhtar et al., Chinese J. Chem., 25, 705-708 (2007); Stanisavljevic et al., Eur. J. Lipid Sci. Technol., 111, 513-518 (2009).

Other methods of forming a biomass isolate or an isolate of one or more anatomical parts from tobacco can be employed. For example, such a method can produce a lipid-containing isolate from a tobacco biomass or anatomical part source. Methods of extracting oil components from plant biomass or one or more anatomical parts are described, for example, in US Pat. Nos. 4,008,210 to Steele et al.; 4,009,290 to Okumori et al.; 4,045,879 to Witte; 4,122,104 to Witte; 4,298,540 to Youn et al.; 4,359,417 to Karnofsky et al.; 4,456,556 to Grimsby; 4,456,557 to Grimsby; 4,466,923 to Friedrich; 4,515,726 to Sullivan; 4,847,106 to Pike et al.; 5,077,071 to Strop; 5,296,621 to Roos et al.; 5,397,571 to Roland et al.; 5,932,095 to Walters et al.; 6,083,729 to Martin et al.; 6,225,483 to Franke; 6,403,126 to Webster et al.; 6,414,172 to Garces et al.; 6,417,157 to Wadsworth et al.; 6,495,175 to Rao et al.; 6,504,085 to Howard; 6,860,998 to Wilde; 7,074,449 to Holley et al.; and 7,156,981 to Wilde et al.; and US Patent Appl. Pub. Nos. 2002/0121628 to Kapila et al.; 2004/0009242 to Krasutsky et al.; 2005/0042347 to Bathurst et al.; 2005/0147722 to Fan et al.; and 2006/0111578 to Arhancet et al.

Components of biomass or of one or more anatomical parts can be subjected to conditions so as to cause those components (whether as part of the biomass or of the one or more anatomical parts or in the form of an isolated component) to undergo chemical transformation. For example, a biomass isolate or an isolate of one or more anatomical parts that has been separated from the biomass or one or more anatomical parts can be treated to cause chemical transformation or can be admixed with other ingredients. Such chemical transformation or modification can result in changes of certain chemical and physical properties of such biomass isolate or isolate of one or more anatomical parts (e.g., sensory attributes of such an isolate). Exemplary chemical modification processes can be carried out by acid/base reaction, hydrolysis, heating (e.g., a thermal treatment where the isolate is subjected to an elevated temperature such as a temperature of at least about 50°C or at least about 75°C or at least about 90°C), and enzymatic treatments (e.g., using hydrolyase, glycosidase, or glucocidase); and as such, components of the isolate can undergo esterification, transesterification, isomeric conversion, acetal formation, acetal decomposition, and the like. Additionally, various isolated lipid components of the biomass or one or more anatomical parts can be subjected to hydrogenation in order to alter the degree of saturation of those components, and hence alter the physical form or behavior of those components.

In one aspect, biomass or one or more anatomical parts can be cold pressed in order to squeeze lipids from the biomass or one or more anatomical parts, and those lipid components are collected and isolated; or alternatively the biomass or one or more anatomical parts can be subjected to solvent extraction using a solvent (e.g., a polar solvent or a non-polar organic solvent), and the resulting extract is collected and the extracted components are isolated. Then, any of the various biomass components or components of one or more anatomical parts may be subjected to enzymatic treatment to form an enzymatically-treated material. The enzymatically-treated material then is subjected to solvent extraction to form a biomass isolate or an isolate of one or more anatomical parts.

In one embodiment, the separating or isolating process comprises freezing harvested biomass or one or more anatomical parts or a portion thereof to form a frozen biomass or anatomical part material, processing the frozen biomass or anatomical part material into a particulate form, subjecting the particulate biomass or anatomical part material to an enzymatic treatment to chemically alter the particulate biomass or anatomical part material, and extracting the particulate biomass or anatomical part material with a solvent to produce a biomass isolate or an isolate of one or more anatomical parts. Exemplary enzymatic treatments include treatment with a glycosidase or a glucosidase.

The biomass or one or more anatomical parts and components or isolates thereof are useful as components for tobacco compositions, particularly tobacco compositions incorporated into smoking articles or smokeless tobacco products. Addition of such components to a tobacco composition can enhance a tobacco composition in a variety of ways, depending on the nature of the biomass or seed isolate and the type of tobacco composition. Exemplary such components can serve to provide flavor and/or aroma to a tobacco product (e.g., composition that alters the sensory characteristics of tobacco compositions or smoke derived therefrom).

The form of biomass isolate or isolate of one or more anatomical parts can vary. Typically, such isolate is in a solid, liquid, or semi-solid or gel form. Biomass or seed isolate can be used in concrete, absolute, or neat form. Such isolate can have a dry particulate form, a waxy form, or a thick paste form. Liquid forms include isolates contained within aqueous or organic solvent carriers.

The biomass or one or more anatomical parts, processed biomass or one or more anatomical parts, and biomass isolates or isolates from one more anatomical parts can be employed in a variety of forms. Biomass or one or more anatomical parts, or an isolate of biomass or of one more anatomical parts, can be employed as a component of processed tobaccos. In one regard, the biomass or one or more anatomical parts, or components thereof, can be employed within a top dressing formulation, or within a casing formulation for application to tobacco strip (e.g., using the types of manners and methods set forth in US Pat. No. 4,819,668 to Shelar). Alternatively, the biomass or one or more anatomical parts, or components thereof, can be employed as an ingredient of a reconstituted tobacco material (e.g., using the types of tobacco reconstitution processes generally set forth in US Pat. Nos. 5,143,097 to Sohn; 5,159,942 to Brinkley et al.; 5,598,868 to Jakob; 5,715,844 to Young; 5,724,998 to Gellatly; and 6,216,706 to Kumar). The biomass or one or more anatomical parts, or components thereof, also can be incorporated into a cigarette filter (e.g., in the filter plug, plug wrap, or tipping paper) or incorporated into cigarette wrapping paper, preferably on the inside surface, during the cigarette manufacturing process. An isolate from biomass or from one or more anatomical parts which isolate has a waxy or smooth texture can be used as a coating for the surface of a formed smokeless tobacco product. An isolate having sticky properties can be used as an adhesive (or component of an adhesive) or binding agent within tobacco products. An isolate having a oily or liquid character can be used as a solvent (e.g., to be used to replace, or act comparable to, a triglyceride type of solvent; or to replace a glycol type of solvent as a humectant or as a carrier for casing components).

The biomass or one or more anatomical parts, processed biomass or one or more anatomical parts, and biomass isolates or isolates from one more anatomical parts can be incorporated into smoking articles. The biomass or one or more anatomical parts, processed biomass or one or more anatomical parts, and biomass isolates or isolates from one more anatomical parts can be admixed with other components that are employed in the manufacture of tobacco products. Exemplary types of further ingredients that can be admixed with the biomass or anatomical part material include flavorants, fillers, binders, pH adjusters, buffering agents, colorants, disintegration aids, antioxidants, humectants and preservatives. Representative tobacco blends, non-tobacco components, and representative cigarettes manufactured therefrom, are set forth in US Pat. Nos. 4,836,224 to Lawson et al.; 4,924,888 to Perfetti et al.; 5,056,537 to Brown et al.; 5,220,930 to Gentry; and 5,360,023 to Blakley et al.; US Pat. Appl. Pub. No. 2002/0000235 to Shafer et al.; and PCT WO 02/37990. Those tobacco materials also can be employed for the manufacture of those types of cigarettes that are described in US Pat. Nos. 4,793,365 to Sensabaugh; 4,917,128 to Clearman et al.; 4,947,974 to Brooks et al.; 4,961,438 to Korte; 4,920,990 to Lawrence et al.; 5,033,483 to Clearman et al.; 5,074,321 to Gentry et al.; 5,105,835 to Drewett et al.; 5,178,167 to Riggs et al.; 5,183,062 to Clearman et al.; 5,211,684 to Shannon et al.; 5,247,949 to Deevi et al.; 5,551,451 to Riggs et al.; 5,285,798 to Banerjee et al.; 5,593,792 to Farrier et al.; 5,595,577 to Bensalem et al.; 5,816,263 to Counts et al.; 5,819,751 to Barnes et al.; 6,095,153 to Beven et al.; 6,311,694 to Nichols et al.; and 6,367,481 to Nichols et al.; US Pat. Appl. Pub. No. 2008/0092912 to Robinson et al.; and PCT WO 97/48294 and PCT WO 98/16125. See, also, those types of commercially marketed cigarettes described Chemical and Biological Studies on New Cigarette Prototypes that Heat Instead of Burn Tobacco, R. J. Reynolds Tobacco Company Monograph (1988) and Inhalation Toxicology, 12:5, p. 1-58 (2000).

The *Nicotiana* biomass or one or more anatomical parts, processed biomass or one or more anatomical parts, and biomass isolates or isolates from one more anatomical parts can be incorporated into smokeless tobacco products, such as loose moist snuff, loose dry snuff, chewing tobacco, pelletized tobacco pieces (e.g., having the shapes of pills, tablets, spheres, coins, beads, obloids or beans), extruded or formed tobacco strips, pieces, rods, cylinders or sticks, finely divided ground powders, finely divided or milled agglomerates of powdered pieces and components, flake-like pieces, molded processed tobacco pieces, pieces of tobacco-containing gum, rolls of tape-like films, readily water-dissolvable or water-dispersible films or strips (e.g., US Pat. App. Pub. No. 2006/0198873 to Chan et al.), or capsule-like materials possessing an outer shell (e.g., a pliable or hard outer shell that can be clear, colorless, translucent or highly colored in nature) and an inner region possessing tobacco or tobacco flavor (e.g., a Newtoniam fluid or a thixotropic fluid incorporating tobacco of some form). Various types of smokeless tobacco products are set forth in US Pat. Nos. 1,376,586 to Schwartz; 3,696,917 to Levi; 4,513,756 to Pittman et al.; 4,528,993 to Sensabaugh, Jr. et al.; 4,624,269 to Story et al.; 4,987,907 to Townsend; 5,092,352 to Sprinkle, III et al.; and 5,387,416 to White et al.; US Pat. App. Pub. Nos. 2005/0244521 to Strickland et al. and 2008/0196730 to Engstrom et al.; PCT WO 04/095959 to Arnarp et al.; PCT WO 05/063060 to Atchley et al.; PCT WO 05/016036 to Bjorkholm; and PCT WO 05/041699 to Quinter et al. See also, the types of smokeless tobacco formulations, ingredients, and processing methodologies set forth in US Pat. Nos. 6,953,040 to Atchley et al. and 7,032,601 to Atchley et al.; US Pat. Appl. Pub. Nos. 2002/0162562 to Williams; 2002/0162563 to Williams; 2003/0070687 to Atchley et al.; 2004/0020503 to Williams, 2005/0178398 to Breslin et al.; 2006/0191548 to Strickland et al.; 2007/0062549 to Holton, Jr. et al.; 2007/0186941 to Holton, Jr. et al.; 2007/0186942 to Strickland et al.; 2008/0029110 to Dube et al.; 2008/0029116 to Robinson et al.; 2008/0029117 to Mua et al.; 2008/0173317 to Robinson et al.; and 2008/0209586 to Neilsen et al.

The residue of biomass or anatomical part material remaining after subjecting biomass or anatomical part material to a separation process (e.g., cold pressing or solvent extraction) and removing some portion of the biomass or one or more anatomical parts can also be incorporated into a tobacco product, including any of the tobacco products mentioned herein with regard to biomass or one or more anatomical parts, or isolates therefrom. For example, a residue remaining after cold pressing biomass or one or more anatomical parts and removing lipid components can be used as a tobacco composition component (e.g., as part of a reconstituted tobacco material), and incorporated into a smoking article or a smokeless tobacco composition. The insoluble pulp residue remaining after solvent extraction of a solvent-soluble portion of a biomass or anatomical part material can likewise be used as a component of a tobacco composition.

Certain isolates, such as triglyceride-containing isolates of biomass or of one or more anatomical parts, can be used as components of capsules used in smoking articles or smokeless tobacco compositions. In particular, triglyceride-containing isolates can be combined with a flavorant and used as a diluting agent or carrier within the internal payload of certain breakable capsules. Typically, such a capsule has an outer wall and an internal liquid, solid, or gel payload. The payload is released upon rupture of the capsule wall. Exemplary capsule-containing tobacco products that could incorporate such isolates are set forth in US Pat. Appl. Pub. Nos. 2004/0261807 to Dube et al.; 2005/0066982 to Clark et al.; 2007/0186941 to Holton et al.; 2008/0302373 to Stokes et al.; and 2009/0050163 to Hartmann et al.

### EXPERIMENTAL

Aspects of the present invention are more fully illustrated by the following example, set forth to illustrate certain aspects of the present invention and not to be construed as limiting thereof.

### Example 1

In connection with the invention it is found that ethanol is readily formed through fermentation of chemically or physically untransformed or transformed biomass or seed from the *Nicotiana* species. Said ethanol is readily isolated. In connection with the invention it is further found that citric acid is readily isolated from physically untransformed or transformed biomass or seed from the *Nicotiana* species. In connection with the invention it is still further found that ethanol so isolated and citric acid so isolated are readily condensed via an esterification reaction to form triethyl citrate.

In an example, biomass is harvested from mature *Nicotiana* plants. The biomass is accumulated by packing it closely enough that anaerobic conditions obtain within the accumulation so formed. Fermentation is allowed to proceed, with the concomitant production of ethanol by various microorganisms.

In an example, harvested biomass is physically processed. The biomass, or parts thereof, is further subdivided into parts or pieces (e.g., the biomass is comminuted, pulverized, milled or ground into pieces or parts that can be characterized as granules, particulates or fine powders). The biomass, or parts thereof, is subjected to external forces or pressure (e.g., by being pressed or subjected to roll treatment). Such processed biomass is found to be suitable feedstock for a fermentative process for the formation of ethanol. Such processed biomass is found to be suitable feedstock for an extractive or other transformative process for the isolation of citric acid.

An article of manufacture is disclosed herein comprising triethyl citrate derived from plants or portions of plants from the *Nicotiana* species by treatment of the plants or portions of plants.

According to the example, conditions for fermentation may be chosen according to the discretion of one skilled in the art, such conditions including such variables as time, temperature, pressure, pH, ionic strength, rate of mixing, agitation, sparging, aeration and so forth.

According to the example, conditions for isolating ethanol from at least a portion of one or more plants from the Nicotiana species may be chosen according to the discretion of one skilled in the art, including conditions for distillation, filtration or other separation, and so forth.

According to the example, conditions for isolating citric acid from at least a portion of one or more plants from the Nicotiana species may be chosen according to the discretion of one skilled in the art, including conditions for distillation, filtration or other separation, and so forth.

According to the example, conditions for reacting ethanol isolated from at least a portion of one or more plants from the *Nicotiana* species with citric acid isolated from at least a portion of one or more plants from the *Nicotiana* species may be chosen according to the discretion of one skilled in the art, such so-called reaction conditions including such variables as time of reaction, temperature pressure, pH, ionic strength, rate of mixing, agitation, sparging, aeration and so forth.

According to the example, any industrially acceptable treatment, or no treatment, of at least a portion of one or more plants from the *Nicotiana* species may be suitable for use of at least a portion of one or more plants from the *Nicotiana* species in a method according to the invention.

## Claims

1. A method for producing triethyl citrate from one or more plants of genus Nicotiana, the method comprising:
isolating an ethanol-containing component from harvested biomass or seed of the *Nicotiana* species by subjecting the harvested biomass or seed or a portion thereof to fermentation followed by cold pressing, solvent extraction, chromatography, distillation, filtration, recrystallization, solvent-solvent partitioning, or a combination thereof to form an isolated ethanol-containing component;
isolating a citric-acid-containing component from harvested biomass or one or more anatomical parts of the *Nicotiana* species by subjecting the harvested biomass or one or more anatomical parts or a portion thereof to cold pressing, solvent extraction, chromatography, distillation, filtration, recrystallization, solvent-solvent partitioning, or a combination thereof to form an isolated citric-acid-containing component;
reacting the ethanol-containing component with the citric-acid-containing component under conditions favoring esterification, thereby forming a triethyl-citrate-containing component.

2. The method of claim 1, wherein the ethanol-containing component is derived from tobacco.

3. The method of claim 2, wherein the ethanol-containing component is derived from tobacco biomass.

4. The method of claim 1, wherein the citric-acid-containing component is derived from tobacco.

5. The method of claim 4, wherein the citric-acid-containing component is derived from tobacco biomass.

6. The method of claim 1, wherein the conditions favoring esterification comprise a temperature between 25 degrees Celsius and 100 degrees Celsius.

7. The method of claim 1 further comprising forming or otherwise further processing the triethyl-citrate-containing fraction into a component of a tobacco-containing article.

8. The method of claim 7, wherein the component is a device for filtration.

9. The method of claim 7, wherein the component is a flavorant.

## Patentansprüche

1. Verfahren zur Herstellung von Triethylcitrat aus einer oder mehreren Pflanzen der Gattung *Nicotiana,* wobei das Verfahren umfasst:
Isolieren einer Ethanol-enthaltenden Komponente aus geernteter Biomasse oder Samen der *Nicotiana*-Spezies durch Unterziehen der geernteten Biomasse oder Samen oder einem Teil davon einer Fermentation, gefolgt von Kaltpressen, Lösungsmittelextraktion, Chromatographie, Destillation, Filtration, Umkristallisation, Lösungsmittel-Lösungsmittel-Verteilung oder einer Kombination davon zur Bildung einer isolierten Ethanol-enthaltenden Komponente;
Isolieren einer Zitronensäure-enthaltenden Komponente aus geernteter Biomasse oder einer oder mehreren anatomischen Anteilen der *Nicotiana*-Spezies durch Unterziehen der geernteten Biomasse oder einer oder mehreren anatomischen Anteilen oder einem Teil davon Kaltpressen, Lösungsmittelextraktion, Chromatographie, Destillation, Filtration, Umkristallisation, Lösungsmittel-Lösungsmittel-Verteilung oder einer Kombination davon zur Bildung einer isolierten Zitronensäure-enthaltenden Komponente;
Umsetzen der Ethanol-enthaltenden Komponente mit der Zitronensäure-enthaltenden Komponente unter die Veresterung begünstigenden Bedingungen, wodurch eine Triethylcitrat-enthaltende Komponente gebildet wird.

2. Verfahren nach Anspruch 1, wobei die Ethanol-enthaltende Komponente von Tabak abgeleitet ist.

3. Verfahren nach Anspruch 2, wobei die Ethanol-enthaltende Komponente von Tabakbiomasse abgeleitet ist.

4. Verfahren nach Anspruch 1, wobei die Zitronensäure-enthaltende Komponente von Tabak abgeleitet ist.

5. Verfahren nach Anspruch 4, wobei die Zitronensäure-enthaltende Komponente von Tabakbiomasse abgeleitet ist.

6. Verfahren nach Anspruch 1, wobei die die Veresterung begünstigenden Bedingungen eine Temperatur zwischen 25 Grad Celsius und 100 Grad Celsius umfassen.

7. Verfahren nach Anspruch 1, weiter umfassend Bilden oder anderweitiges weiteres Verarbeiten der Triethylcitrat-enthaltenden Fraktion zu einer Komponente eines Tabakenthaltenden Gegenstandes.

8. Verfahren nach Anspruch 7, wobei die Komponente eine Vorrichtung zur Filtration ist.

9. Verfahren nach Anspruch 7, wobei die Komponente ein Geschmacksstoff ist.

## Revendications

1. Procédé de production de citrate de triéthyle à partir d'une ou de plusieurs plantes du genre Nicotiana, le procédé comprenant :
l'isolement d'un composant contenant de l'éthanol à partir d'une biomasse ou de graines récoltées de l'espèce *Nicotiana* en soumettant la biomasse ou les graines récoltées ou une portion de celles-ci à une fermentation suivie d'un pressage à froid, d'une extraction par solvant, d'une chromatographie, d'une distillation, d'une filtration, d'une recristallisation, d'une séparation solvant-solvant, ou d'une combinaison de celles-ci pour former un composant contenant de l'éthanol isolé ;
l'isolement d'un composant contenant de l'acide citrique à partir de biomasse ou d'une ou plusieurs parties anatomiques récoltées de l'espèce *Nicotiana* en soumettant la biomasse ou les une ou plusieurs parties anatomiques récoltées ou une portion de celles-ci à un pressage à froid, une extraction par solvant, une chromatographie, une distillation, une filtration, une recristallisation, une séparation solvant-solvant, ou une combinaison de celles-ci pour former un composant contenant de l'acide citrique isolé ;
la réaction du composant contenant de l'éthanol avec le composant contenant de l'acide citrique dans des conditions favorisant l'estérification, en formant ainsi un composant contenant du citrate de triéthyle.

2. Procédé selon la revendication 1, dans lequel le composant contenant de l'éthanol est dérivé du tabac.

3. Procédé selon la revendication 2, dans lequel le composant contenant de l'éthanol est dérivé d'une biomasse de tabac.

4. Procédé selon la revendication 1, dans lequel le composant contenant de l'acide citrique est dérivé du tabac.

5. Procédé selon la revendication 4, dans lequel le composant contenant de l'acide citrique est dérivé d'une biomasse de tabac.

6. Procédé selon la revendication 1, dans lequel les conditions favorisant l'estérification comprennent une température entre 25 degrés Celsius et 100 degrés Celsius.

7. Procédé selon la revendication 1 comprenant en outre la formation ou sinon en outre la transformation de la fraction contenant du citrate de triéthyle en un composé d'un article contenant du tabac.

8. Procédé selon la revendication 7, dans lequel le composant est un dispositif pour filtration.

9. Procédé selon la revendication 7, dans lequel le composant est un aromatisant.
